# EUROPEAN PATENT APPLICATION

(11) **EP 4 067 904 A1**
(43) Date of publication of application: **05.10.2022**
(21) Application number: 20893719.3
(22) Date of filing: 27.11.2020
(51) Int. Cl.: G01N 33/53, G01N 33/543

(54) **ANALYTE MEASUREMENT METHOD USING IMMUNE RESPONSE AND MEASUREMENT REAGENT**

(30) Priority: 29.11.2019 JP 2019216967
(71) Applicant: Sekisui Medical Co., Ltd., Tokyo 103-0027 (JP)
(72) Inventor: ASAI Tomohide, Tokyo 103-0027 (JP); TANAKA Yoshihiro, Tokyo 103-0027 (JP); SUZUKI Ikumi, Tokyo 103-0027 (JP); SEO Satoko, Tokyo 103-0027 (JP); USUI Tomoyuki, Tokyo 103-0027 (JP); SHIMIZU Tomo, Tokyo 103-0027 (JP); MIYAZAKI Osamu, Tokyo 103-0027 (JP)
(74) Representative: Hoffmann Eitle
(86) International application number: PCT/JP2020/044248
(87) International publication number: WO 2021/107105

(57) **Abstract**

A problem to be solved by the invention is to improve the sensitivity of measurement of an analyte in a sample by a sandwich immunoassay. The sensitivity can be improved significantly in a sandwich immunoassay using a first antibody and a second antibody when one or both of the first antibody and the second antibody are a mixture of a monoclonal antibody recognizing a linear epitope and a monoclonal antibody recognizing a conformational epitope.

## Description

### TECHNICAL FIELD

The present invention relates to a measurement method and a measurement reagent for an analyte using immunoreaction. Further specifically, the invention relates to a measurement method and a measurement reagent for an analyte for a sandwich immunoassay using a first antibody and a second antibody in which an antibody recognizing a linear epitope and an antibody recognizing a conformational epitope are mixed and used as the first antibody and/or the second antibody.

### BACKGROUND ART

PIVKA-II is a protein that is prothrombin, a blood coagulation factor II, but without coagulation factor activity; PIVKA-II is also called abnormal prothrombin. Prothrombin is synthesized in the liver and requires the conversion of glutamic acid (Glu) residues into γ-carboxyglutamic acid (Gla) residues by vitamin K-dependent γ-glutamyl carboxylase in the production process. Although normal prothrombin has 10 Gla residues around the N-terminus, all or a part of the 10 residues are not converted into Gla and remain as Glu residues in PIVKA-II. PIVKA-II was originally found in the blood of patients with vitamin K deficiency or patients receiving a vitamin K antagonist. Because the blood concentration increases with hepatoma, PIVKA-II has been recently measured as a tumor marker for hepatoma. PIVKA-II is the abbreviation for protein induced by Vitamin K absence or antagonists-II and is also called des-γ-carboxy prothrombin (DCP). (Reference Documents: Weitz, I. C., and Liebman, H. A.;(1993) Hepatology 18, 990-997, Suzuki M, Shiraha H, Fujikawa T, Takaoka N, Ueda N, Nakanishi Y, Koike K, Takaki A, Shiratori Y; J Biol Chem. 2005 Feb 25;280(8):6409-15 and A. Nakao, A. Virji, Y. Iwaki, B. Carr, S. Iwatsuki, and E. Starzl; Hepatogastroenterology. 1991 October; 38(5): 450-453)

As a reagent for specifically measuring PIVKA-II in a sample, Picolumi (registered trademark) PIVKA-II MONO kit (Sekisui Medical Co., Ltd.) is sold. The measurement of the product is based on electrochemiluminescence immunoassay (ECLIA) by the sandwich method using beads on which an anti-PIVKA-II mouse monoclonal antibody is bound as the solid phase and an anti-prothrombin mouse monoclonal antibody labeled with a ruthenium (Ru) complex which emits light through an electrochemical change. When the anti-PIVKA-II mouse monoclonal antibody-bound beads and an analyte are reacted in the first reaction, the PIVKA-II in the analyte binds to the anti-PIVKA-II mouse monoclonal antibody on the beads. Next, as the second reaction, after washing the beads, the ruthenium-labeled anti-prothrombin mouse monoclonal antibody is reacted with the PIVKA-II bound to the beads and thus bound to form a sandwich structure. After further washing the beads, when electrical energy is applied on electrodes, the ruthenium complex emits light according to the amount of the ruthenium-labeled anti-prothrombin mouse monoclonal antibody which is bound to the beads through the PIVKA-II. By measuring the luminescence, the PIVKA-II amount in the analyte can be accurately measured (see the documents attached to Picolumi (registered trademark) PIVKA-II MONO kit, fifth edition). Picolumi (registered trademark) PIVKA-II MONO kit has a measurement range of 10 to 75,000 mAU/mL and is characterized by the high sensitivity and the wide measurement range.

On the other hand, the reference upper limit of serum or plasma PIVKA-II value for healthy adults is 28 mAU/mL, and the cut-off value calculated from the sensitivity and the specificity in hepatoma, hepatic cirrhosis and chronic hepatitis patients is set at 40 mAU/mL. Accordingly, the cut-off value is around the lower end of the wide measurement range of Picolumi (registered trademark) PIVKA-II MONO kit, and thus there are needs for development of a measurement method with higher sensitivity.

JP-A-6-113830 discloses that use of a mixture of more than one monoclonal antibody which specifically reacts with human hemoglobin and which is bound to a carrier enables easy human hemoglobin-specific agglutination reaction with high accuracy, specific measurement of human hemoglobin in a biological sample with high sensitivity, application to measurement of hemoglobin in human stool or urine and application to a diagnosis of colon cancer, renal disease or the like.

The present inventors have extensively worked on the development of a highly sensitive measurement method for PIVKA-II and realized that, when more than one labeled monoclonal antibody was used in a sandwich immunoassay using a first antibody(s) and a second antibody(s), there were combinations in which the sensitivity improved significantly and combinations in which the sensitivity did not improve as compared to the cases using the antibodies individually.

The present inventors have determined the epitopes of the antibodies and surprisingly found that a combination of antibodies with significantly improved sensitivity was a combination of a monoclonal antibody recognizing a linear epitope and a monoclonal antibody recognizing a conformational epitope. The invention has been thus completed.

### CITATION LIST

### PATENT LITERATURE

[Patent Document 1] JP H6-113830 A
[Patent Document 2] JP H5-043357 A

### SUMMARY OF INVENTION

### TECHNICAL PROBLEM

A problem to be solved by the invention is to improve the sensitivity of measurement of an analyte in a sample by a sandwich immunoassay. In another aspect, a problem to be solved by the invention is to improve the sensitivity of measurement especially when the cut-off value of the analyte is around the lower limit of the measurement range of the measurement method.

### SOLUTION TO PROBLEM

As a result of extensive examination to solve the problems, the present inventors have found that the sensitivity can be improved significantly in a sandwich immunoassay using a first antibody(s) (singular or plural, the same applies below) and a second antibody(s) (singular or plural, the same applies below) when one or both of the first antibody(s) and the second antibody(s) are a mixture of a monoclonal antibody recognizing a linear epitope and a monoclonal antibody recognizing a conformational epitope. The invention has been thus completed. That is, the invention has the following structures.

### [Embodiment 1]

A sandwich immunoassay method for measuring an analyte using a first antibody(s) and a second antibody(s), wherein one or both of the first antibody(s) and the second antibody(s) are a mixture of a monoclonal antibody recognizing a linear epitope and a monoclonal antibody recognizing a conformational epitope.

### [Embodiment 2]

A sandwich immunoassay method for measuring an analyte in a sample including the following steps:
a step of providing a sample;
a step of bringing a first antibody(s) into contact with the sample and thereby providing a first reactant;
a step of optionally recovering a first antibody-analyte complex contained in the first reactant;
a step of bringing a second antibody(s) into contact with the first reactant or the first antibody-analyte complex and thereby providing a second reactant;
a step of recovering a first antibody-analyte-second antibody complex contained in the second reactant; and
a step of measuring the analyte in the sample by measuring the signal of a label of the recovered first antibody-analyte-second antibody complex;
wherein the first antibody(s) and the second antibody(s) are antibodies for different epitopes of the analyte, and
one or both of the first antibody(s) and the second antibody(s) are a mixture of a monoclonal antibody recognizing a linear epitope and a monoclonal antibody recognizing a conformational epitope.

### [Embodiment 3]

The method according to Embodiment 1 or 2, wherein the first antibody(s) is an immobilized antibody.

### [Embodiment 4]

The method according to Embodiment 1 or 2, wherein the second antibody(s) is an immobilized antibody.

### [Embodiment 5]

The method according to any of Embodiments 1 to 3, wherein the second antibody(s) is a labeled antibody.

### [Embodiment 6]

The method according to any of Embodiments 1, 2 and 4, wherein the first antibody(s) is a labeled antibody.

### [Embodiment 7]

The method according to any of Embodiments 1 to 6, wherein the analyte is PIVKA-II.

### [Embodiment 8]

The method according to any of Embodiments 1 to 7, wherein the monoclonal antibody recognizing a linear epitope is an antibody which recognizes a peptide having the sequence ((E/y)AF(E/y)AL(E/y)SSTATDVFWAKY) of the 26th to 44th amino acids of PIVKA-II or a peptide having the sequence ((E/y)AL(E/y)SSTATDVFWAKY) of the 29th to 44th amino acids of PIVKA-II.

### [Embodiment 9]

The method according to any of Embodiments 1 to 8, wherein the monoclonal antibody recognizing a conformational epitope is an antibody which recognizes the conformation of the amino acid sequence (SSTATDVFWAKYT) of the 33rd to 45th residues, the amino acid sequence (TATDVFWAKYT) of the 35th to 45th residues or the amino acid sequence (TATDVFWA) of the 35th to 42nd residues of PIVKA-II.

### [Embodiment 10]

A sandwich immunoassay reagent for measuring an analyte comprising a first antibody(s) and a second antibody(s), wherein one or both of the first antibody(s) and the second antibody(s) are a mixture of a monoclonal antibody recognizing a linear epitope and a monoclonal antibody recognizing a conformational epitope.

### [Embodiment 11]

The method according to any of Embodiments 1 to 9 or the reagent according to Embodiment 10, wherein the mixture is a mixture of two kinds of monoclonal antibody derived from two kinds of hybridoma.

### [Embodiment 12]

The method according to any of Embodiments 1 to 9 or the reagent according to Embodiment 10, wherein the monoclonal antibody recognizing a linear epitope is a monoclonal antibody derived from a first hybridoma, and the monoclonal antibody recognizing a conformational epitope is a monoclonal antibody derived from a second hybridoma.

### [Embodiment 13]

The method according to any of Embodiments 1 to 9 or the reagent according to Embodiment 10, wherein the mixture includes two kinds of immunoglobulin molecule.

### [Embodiment 14]

The method according to any of Embodiments 1 to 9 or the reagent according to Embodiment 10, wherein the monoclonal antibody recognizing a linear epitope includes a first immunoglobulin molecule, and the monoclonal antibody recognizing a conformational epitope includes a second immunoglobulin molecule.

### ADVANTAGEOUS EFFECTS OF INVENTION

According to the invention, more precise measurement of an analyte becomes possible. Moreover, a diagnosis or support of a diagnosis of disease-clinical conditions using the analyte as a marker can be made with higher reliability. In particular, when the cut-off value of the analyte is around the lower limit of the measurement range of the existing sandwich immunoassay using a first antibody(s) and a second antibody(s), a diagnosis or support of a diagnosis of disease-clinical conditions can be made with higher reliability.

### BRIEF DESCRIPTION OF DRAWINGS

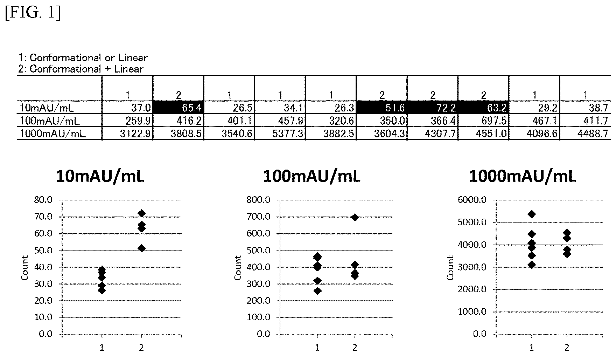

[Fig. 1] A plot of the measurement values (counts) of the standard antigen solutions of 10 mAU/mL, 100 mAU/mL and 1000 mAU/mL for the case (1) in which a monoclonal antibody recognizing a linear epitope or a monoclonal antibody recognizing a conformational epitope alone was used as a Ru-labeled anti-Fl antibody and the case (2) in which a mixture of a monoclonal antibody recognizing a linear epitope and a monoclonal antibody recognizing a conformational epitope was used as a Ru-labeled anti-F1 antibody.

### DESCRIPTION OF EMBODIMENTS

### (Definition)

First antibody/second antibody: In the present specification, the term "first antibody" means one of the antibodies in the sandwich structure (antibody-analyte-antibody complex) formed in the sandwich immunoassay, and the term "second antibody" means the other antibody forming the complex with the first antibody(s) through the analyte. That is, in the sandwich structure, the analyte is sandwiched between the first antibody(s) and the second antibody(s). The "first antibody" used in the specification of the present application means a first monoclonal antibody in an aspect. In this case, the "first antibody" is not a mixture of more than one monoclonal antibody. In another aspect, however, the term "first antibody" means a first set of monoclonal antibodies. In this case, the "first antibody" is a mixture of more than one monoclonal antibody. The same applies to the term "second antibody". It would be obvious to one skilled in the art that the first antibody(s) and the second antibody(s) need to recognize different epitopes (the antibodies do not inhibit the binding of each other) to form the sandwich structure. On the other hand, when the first antibody(s) (or the second antibody(s)) is a mixture of more than one monoclonal antibody, the epitopes of the monoclonal antibodies do not have to be different and may be the same or partially overlap.

Sample: The "sample" used in the specification of the present application means a biological sample of a mammal, preferably of a human. The biological sample may be any sample as long as the sample possibly contains prothrombin (for example, a sample derived from a tissue expressing prothrombin, body fluid with prothrombin circulation and the like), but blood, serum, plasma or lymph fluid is preferable.

Analyte: The subject to be measured is not particularly limited as long as the subject is a molecule to which the antigen-antibody reaction can be applied, and examples thereof include PIVKA-II, prothrombin, CRP (C-reactive protein), Lp (a), MMP3 (matrix metalloproteinase-3), collagen type IV, PSA (prostate-specific antigen), BNP (brain natriuretic peptide), insulin, microalbumin, cystatin C, an antiphospholipid antibody, an anti-treponema pallidum antibody, FDP (fibrin and fibrinogen degradation product), D-dimer, SF (soluble fibrin), TAT (thrombin-antithrombin III complex), factor XIII, pepsinogen I-II and the like. In this regard, however, a low molecular weight compound or a short peptide molecule which cannot sense a linear epitope or a conformational epitope cannot be used as the analyte due to the principle of the invention.

PIVKA-II: The "PIVKA-II" used in the specification of the present application means PIVKA-II of a mammal, preferably of a human.

Prothrombin: In the specification of the present application, normal and abnormal prothrombin are collectively called "prothrombin". In the specification of the present application, the "abnormal prothrombin" means PIVKA-II, and the "normal prothrombin" means prothrombin other than PIVKA-II.

Sandwich immunoassay: The term "sandwich immunoassay" used in the specification of the present application is used with the meanings known to one skilled in the art. More specifically, the sandwich immunoassay means an immunoassay which enables specific measurement of an analyte through formation of a so-called "sandwich" structure through binding of both of a first antibody(s) and a second antibody(s) to the analyte. The "sandwich immunoassay" known to one skilled in the art is electrochemiluminescence immunoassay (ECLIA), enzyme-linked immunosorbent assay (ELISA), latex turbidimetric immunoassay (LTIA) or lateral flow immunoassay.

Linear epitope: The "linear epitope" used in the present specification is a part of an antigen which an antibody recognizes or binds to and means one formed by the primary structure of a protein or a polypeptide. In the present specification, the terms such as "linear" structure and "primary" structure are used as synonyms.

Conformational epitope: The "conformational epitope (higher order structure epitope)" used in the present specification is a part of an antigen which an antibody recognizes or binds to and means a part in an antigen having a higher order structure in the native state to which an antibody which does not react with a linear peptide that is an extracted primary structure of the protein reacts. In the present specification, the term "conformation" is used as the synonym of the term "higher order structure".

Monoclonal antibody: In the present specification, the term "a/the monoclonal antibody(s)" means a set of immunoglobulin molecules of a single molecular kind having a same structure. The "monoclonal antibody" used in the specification of the present application may be the antibody itself but may also be a fragment having the avidity to the antigen such as a Fab fragment and a F(ab')₂ fragment. The monoclonal antibody can be a classical monoclonal antibody obtained through immunization of a non-human animal with an antigen, a monoclonal antibody obtained by gene recombination, gene immunization or the like, and the method for obtaining the monoclonal antibody is not limited. The antibody may be bound to a known labeling substance such as a ruthenium (Ru) complex, peroxidase, alkaline phosphatase, biotin, a colloidal metal and FITC. The "reagent containing an antibody" may contain an appropriate salt, a buffer, a preservative, a surfactant, a reducing agent, a cryoprotectant or the like. Here, although use of two kinds of monoclonal antibody is essential in the invention, this does not intend to exclude the use of third kind or more. Moreover, the case in which the two kinds of monoclonal antibody, which are immobilized on carrier particles for use, are not immobilized on carrier particles and further formulated in the free form is not excluded.

Immobilization: In the specification of the present application, the term "immobilization" is used with the same meanings as those of "fixation" or "sensitization".

Carrier particles: Examples of the carrier particles (insoluble carrier) used in the invention include magnetic beads, organic polymer powder, inorganic substance powder, a microorganism, hemocytes, a cell fragment and the like.

Examples of the organic polymer powder include natural polymer powder such as insoluble agarose, cellulose and insoluble dextran, synthetic polymer powder such as polystyrene, styrene-styrene sulfonate copolymer, acrylonitrile-butadiene-styrene copolymer, vinyl chloride-acrylic acid ester copolymer and vinyl acetate-acrylic acid ester copolymer and the like, and in particular, latex particles obtained by uniformly suspending synthetic polymer powder are preferable.

Examples of the inorganic substance powder include metal pieces of gold, titanium, iron, nickel and the like, silica, alumina, carbon powder and the like.

Regarding the average particle size of the insoluble carrier, those having an average particle size of 0.05 to 10.0 µm are generally used. The particle sizes and the materials of the two kinds of carrier particle carrying the two kinds of monoclonal antibody may be the same or different.

Bringing into contact: That the carrier particles are "brought into contact" with the biological sample in the specification of the present application means that the particles and the sample are mixed in any of the forms of solid, aqueous solution and suspension.

### EXAMPLES

### (Experimental Materials and Methods)

### <Monoclonal Antibody (Anti-PIVKA-II Antibody): MU-3 Antibody>

### (1) Production Method

As an anti-PIVKA-II monoclonal antibody (MU-3 antibody), an antibody produced by the method described in Example 1 of JP-B-5-43357 itself was used.

The antibody production method described in Example 1 of JP-B-5-43357 is cited below with partial omission.

"B. BaSO₄ and BaCO₃ were added each at 100 mg/mL to plasma of an individual taking warfarin, and after stirring for 120 minutes, normal prothrombin was removed by adsorption. Next, ion exchange was conducted after adding DE-52 cellulose, and the resultant was loaded to an affinity column using a monoclonal antibody for a part common to both of normal prothrombin and PIVKA-II and eluted with 4M guanidine hydrochloride. The eluate was dialyzed and concentrated to purify PIVKA-II. The obtained PIVKA-II (50 µg) together with the same volume of complete Freund's adjuvant was intraperitoneally administered to BALB/C mice (female, 4 weeks old), and PIVKA-II (15 µg) was administered to the tail vein after two weeks. The splenic cells were taken out after three days and fused with a tumor cell line P3U1. The cells were fused by the method of Watanabe *et al.* using polyethylene glycol 4000. Next, cloning was conducted three times using a 96-well microplate by the limiting dilution analysis. For the assay for the cloning, decarboxylated human prothrombin of A above and eventually native PIVKA-II were used. The cell lines of the antibody-producing hybridomas established by the cloning were given the identification symbols of..., MU-3, ... A monoclonal anti-PIVKA-II antibody was obtained from cell line MU-3 by a general method."

As described in the cited part, the screening for anti-PIVKA-II antibodies produced by hybridomas obtained by immunization with PIVKA-II was conducted through an assay for cloning antibody-producing hybridomas using decarboxylated human prothrombin and eventually native PIVKA-II or the like.

The epitope site of MU-3 antibody has been identified-confirmed to be the "decarboxyl peptide site of the 13th to 23rd positions of the amino acid sequence of prothrombin" through detailed research on the binding potential using peptide fragments having various lengths synthesized based on the Gla region amino acid sequence of PIVKA-II (JP-A-7-20127). Accordingly, by appropriately combining the peptide fragments having various lengths synthesized based on the Gla region amino acid sequence of PIVKA-II described in JP-A-7-20127 or the like in addition to native PIVKA-II and decarboxylated human prothrombin described in JP-B-5-43357 and comparing-determining the binding potential of the antibodies to PIVKA-II and to a substance other than PIVKA-II, screening for anti-PIVKA-II antibodies can be conducted. As a result, a new anti-PIVKA-II antibody other than MU-3 antibody can also be obtained. In the present specification, all the contents described in JP-B-5-43357 and JP-A-7-20127 are included by reference.

Furthermore, anti-PIVKA-II antibodies other than MU-3 are also known. For example, 2G4 antibody described in JP-A-9-43237, the antibodies described in JP-A-60-60557, the antibodies described in JP-A-7-313186 and the like are believed to be able to be used as the anti-PIVKA-II antibody of the invention.

### <Monoclonal Antibodies (Anti-Prothrombin F1 Region Antibodies): Antibody 242202, Antibody 242203, Antibody 242205 and Antibody 242206>

### (1) Production Method

### i) Preparation of Hybridomas

Emulsions were obtained by mixing and emulsifying purified silkwormexpressed PIVKA-II (manufactured by Ozeki Corporation) or a synthetic peptide (the amino acid sequence of the 29-47th positions) (SEQ ID NO:1) and complete Freund's adjuvant (manufactured by GIBCO) at 1:1 and subcutaneously administered to 8-weekold female BALB/C mice (manufactured by Charles River Laboratories Japan, Inc) at a dose of 20 µg/100 µL five times at intervals of two weeks. The spleens were extracted three days after the final immunization. The splenic cells obtained from an extracted spleen and myeloma cells SP2/O-Ag14 were mixed at a ratio of 10 to 1, and the cells were fused in the presence of 50 wt% polyethylene glycol 1540 (manufactured by Wako Pure Chemical Industries, Ltd.). The fused cells were suspended in HAT medium in such a manner that the cell count of the splenic cells became 2.5×10⁶ cells/mL and dispensed to a 96-well culture plate (manufactured by CORNING) at 0.2 mL. This was cultured in a 5 vol% CO₂ incubator at 37°C. After approximately two weeks, the culture supernatants of the wells in which hybridomas grew were assessed by the ELISA method shown below, and hybridomas producing an antibody reacting with PIVKA-II were selected.

Specifically, first, 1 µg/mL of PIVKA-II prepared by the method of Bajah *et al.* (the method described in S. Paul Bajah, Paul A. Price, and William A. Russell, Decarboxylation of γ-Carboxyglutamic Acid Residues in Human Prothrombin, Journal of Biological Chemistry 257(7):3726, 1982) was immobilized on a 96-well ELISA plate (manufactured by NUNC). After reacting the culture supernatants therewith, a peroxidase-labeled goat anti-mouse IgG antibody (jackson Immuno Research) was reacted, and then the colors were developed by adding OPD coloring solution. After stopping the coloration by adding a stop solution, hybridoma strains which reacted with PIVKA-II were selected by measuring with a microplate reader (Abs. 492 nm). From the selected hybridoma strains, hybridomas 242202, 242203, 242205 and 242206, which brought about excellent results when a sandwich immunoassay system was constructed in combination with MU-3 antibody, were obtained. Hybridomas 242202, 242203 and 242205 were obtained from mice immunized with PIVKA-II, and hybridoma 242206 was obtained from a mouse immunized with the synthetic peptide.

### ii) Preparation of Monoclonal Antibodies

Monoclonal antibody 242202 (antibody 202), monoclonal antibody 242203 (antibody 203), monoclonal antibody 242205 (antibody 205), and monoclonal antibody 242206 (antibody 206) were prepared from hybridoma 242202, hybridoma 242203, hybridoma 242205, and hybridoma 242206, respectively, by the following method.

Each hybridoma was seeded in a serum-free medium (S-Clone SF-B) at 1×10⁵/4 mL in a flask and then cultured to 1 L, and a culture solution containing the antibody was thus obtained. The culture solution was centrifuged, and a supernatant was obtained. After mixing the supernatant with the equivalent amount of an adsorption buffer solution (3 mol/L NaCl-1.5 mol/L Glycine-NaOH, pH8.5), the mixture was filtered. The filtrate was passed through a protein A column (HiTrap rProteinA FF, manufactured by GE Healthcare Japan) which was equilibrated with the adsorption buffer solution, and the antibody was adsorbed on the column. Then, the monoclonal antibody was purified by elution with 0.1 mol/L citrate buffer solution (pH3.0).

### (2) Determination of Monoclonal Antibody Epitopes

### i) Analysis with Linear Peptides

From a preliminary analysis, it was elucidated that all the antibodies produced by hybridomas 242202, 242203, 242205 and 242206 bind to the Fragment 1 (F1) region of PIVKA-II. To identify the epitopes of the antibodies, screening of the antibodies based on the reactivity to peptides corresponding to the sequence of the 26th to 47th amino acids of PIVKA-II was conducted. The region is the region from the C-terminus of the Gla domain in the F1 region of PIVKA-II to just before the thrombin cleavage site (the 51 st position ↓, the 52nd position). The region after the 33rd amino acid of PIVKA-II has the same amino acid sequence as that of the corresponding region of prothrombin. For the screening, peptides L1 to L7 (SEQ ID NOs: 2 to 8) purchased from Toray Industries, Inc. were used. The N-terminus of each peptide was biotinylated.

The peptides were dissolved in PBS to 10 nM. DynabeadsTM M-270 Streptavidin were suspended in the peptide solutions, and 10 mg/mL solutions were thus prepared. After leaving to stand still for 10 minutes, the beads were washed three times with the Ru diluent (1) described below to obtain peptide-immobilized magnetic beads. The beads were diluted to 1 mg/mL, and peptide-immobilized magnetic bead solutions were thus obtained.

To 1 mL of an anti-mouse IgG Fab antibody (prepared at 1 mg/mL), 68 µL (10 mg/mL) of a Ru complex compound of succinimide group-modified tri-dipyridyl ruthenium was added, and the reaction was advanced under stirring at room temperature for 30 minutes. The reaction was stopped by adding 50 µL of 2 mol/L glycine, and the reaction was further advanced under stirring at room temperature for 10 minutes. In the end, the sample was loaded to sephadex G-25 (equilibrated with 10 mmol/L phosphate buffer solution) to collect a Ru-bound protein fraction, and a Ru-labeled anti-mouse IgG Fab antibody was thus prepared (sometimes abbreviated to "Ru-labeled anti-mouse antibody" below). The obtained Ru-labeled anti-mouse IgG Fab antibody was diluted with the Ru diluent (1) to a final concentration of 2 µg/mL before use. The composition of the Ru diluent (1) is shown below.

### Composition of Ru diluent (1): 50 mM HEPES, 1 mM EDTA-4Na, 0.05% NaN3, 1% BSA, 0.1% Tween20, pH7.8

Using the prepared peptide-immobilized magnetic bead solutions, the Ru-labeled anti-mouse antibody solution and an automated ECLIA assay device "Picolumi III" (manufactured by Sekisui Medical Co., Ltd.), the epitopes of the obtained monoclonal antibodies were analyzed by electrochemiluminescence immunoassay.

To 100 µL of the Ru diluent (1), 20 µL of a monoclonal antibody solution (0.5 µg/mL) and 12.5 µL of the peptide-immobilized magnetic beads were added, and the reaction was advanced for nine minutes. After reaction for nine minutes after adding 200 µL of the Ru-labeled anti-mouse antibody solution which was diluted with the Ru diluent (1) to 0.2 mg/mL, the magnetic particles were washed, and measurement was made. The analysis results are summarized in Table 1 below, which shows a count value less than 2000 with -, a value of 2000 or more and less than 10000 with +-, a value of 10000 or more and less than 20000 with +, a value of 20000 or more and less than 30000 with ++ and a value of 30000 or more with +++.

**[Table 1]**

| Peptide Number | PIVKA-II Amino Acid Sequence | | | | | | | | | | | | | | | | | | | | | | Anti-PIVKA-II Antibody | | | |
|---|---|---|---|---|---|---|---|---|---|---|---|---|---|---|---|---|---|---|---|---|---|---|---|---|---|---|
| | 26 | 27 | 28 | 29 | 30 | 31 | 32 | 33 | 34 | 35 | 36 | 37 | 38 | 39 | 40 | 41 | 42 | 43 | 44 | 45 | 46 | 47 | 242202 | 242203 | 242205 | 242206 |
| L1 | E | A | F | E | A | L | E | S | S | T | A | T | D | V | F | W | A | K | Y | | | | ± | - | +++ | +++ |
| L2 | E | A | F | E | A | L | γ | S | S | T | A | T | D | V | F | W | A | K | Y | | | | - | - | ++ | +++ |
| L3 | E | A | F | γ | A | L | E | S | S | T | A | T | D | V | F | W | A | K | Y | | | | - | - | ++ | +++ |
| L4 | E | A | F | γ | A | L | γ | S | S | T | A | T | D | V | F | W | A | K | Y | | | | - | - | ++ | +++ |
| L5 | | | | E | A | L | E | S | S | T | A | T | D | V | F | W | A | K | Y | T | A | C | ± | - | ++ | +++ |
| L6 | | | | | | | | | | T | A | T | D | V | F | W | A | K | Y | | | | - | - | ++ | - |
| L7 | | | | | | | | | | T | A | T | D | V | F | W | A | K | Y | T | A | C | - | - | + | - |

| | | | | | | | | | | | | | | | | | | | | | | | | | | |
|---|---|---|---|---|---|---|---|---|---|---|---|---|---|---|---|---|---|---|---|---|---|---|---|---|---|---|
| E: Glu residue, *γ*: Gla residue | | | | | | | | | | | | | | | | | | | | | | | | | | |

From the analysis results, it was confirmed that antibody 205 reacts with the peptide sequence of the 26-44th positions of PIVKA-II and that antibody 206 reacts with the peptide sequence of the 29-44th positions of PIVKA-II. No strong binding was observed between antibody 202 or antibody 203 and L1 to L7 peptides in Table 1.

### ii) Analysis with Conformation-Added Peptides

The epitopes of antibody 202 and antibody 203 were analyzed using the PEPperMAP (registered trademark) Conformational Epitope Mapping service of PEPperPRINT GmbH.

Amino acid sequences were designed by shifting consecutive 7, 10 and 13 amino acid residues by one amino acid residue from the N-terminus on the sequence obtained by adding GSGSGSG sequence to the N-terminus and the C-terminus of the sequence of the Fragment 1 region of PIVKA-II, and 480 kinds of peptide having the amino acid sequences were synthesized. Cysteine was added to the C-terminus of each peptide, and an appropriate functional group which reacts with the cysteine was added to the N-terminus. The group of synthesized peptides were cyclized through formation of a thioester bond between the cysteine and the functional group to add a higher order structure and then printed on a microarray. Rockland blocking buffer MB-070 was added to the microarray for blocking for 30 minutes, and antibody 202 and antibody 203 which were prepared at 1, 10 and 100 µg/mL using an incubation buffer (a solution obtained by 10-fold dilution of Rockland blocking buffer MB-070 with PBS, 0.005% Tween20, pH7.4) were reacted at 4°C for 16 hours under shaking at 140 rpm. After washing with PBS, 0.005% Tween20, pH7.4 for 10 seconds twice, Goat anti-mouse IgG(H+L) DyLight680 which was diluted 5000-fold with an incubation buffer was reacted at room temperature for 45 minutes, and measurement was made with LI-COR Odyssey Imaging System with the settings of offset of 0.65 mm and resolution of 21 µm.

As a result, antibody 202 bound most strongly to the cyclic peptide SSTATDVFWAKYT (peptide No. 40), and antibody 203 bound most strongly to the cyclic peptide STATDVFWAKYTA (peptide No. 41). It was thus shown that antibody 202 and antibody 203 recognize the conformational epitope of the 35-45th positions of PIVKA-II. More specifically, it was shown that antibody 202 is an antibody recognizing the conformational epitope of the 35-44th positions of PIVKA-II and that antibody 203 is an antibody recognizing the conformational epitope of the 36-45th positions of PIVKA-II/prothrombin. Moreover, it was suggested that both antibodies may recognize the TATDVFWA motif at the 3 5-42nd positions. The region forms a loop, which is different from the loop corresponding to the sequence of PIVKA-II recognized by MU-3 antibody (the 13th to 23rd positions), and an α-helix, in the crystalline structure of prothrombin (PDB ID: 6BJR(http://www.rcsb.org/pdb/explore/explore.do?structureId=6BJR)). Accordingly, it is suggested that antibody 202 and antibody 203 recognize the conformational epitope of the loop and/or the α-helix formed by the amino acids at the 33-45th positions at the C-terminus of the Gla domain (the 8-45th positions) of PIVKA-II.

### <Preparation of MU-3 Monoclonal Antibody-Immobilized Magnetic Bead Solution>

As resin particles, 2.0 g of micropearl EX-003 (particle size of 3.01 µm, CV 3.1%, manufactured by Sekisui Chemical Co., Ltd.) was ultrasonically dispersed in 40.0 g of ion exchanged water, and a core particle dispersion was obtained. Subsequently, 4.0 mL of magnetic fluid EMG707 (manufactured by Ferrotec Corporation) was added under stirring with irradiation with ultrasonic waves and further ultrasonically dispersed for 30 minutes. The obtained dispersion was filtered and washed with ion exchanged water to remove the excessive magnetic fluid, and magnetically responsive beads were thus obtained.

After ultrasonically dispersing 1.0 g of the magnetically responsive beads in 400 g of ethanol, 10 mL of 28% aqueous ammonia solution (manufactured by Nacalai Tesque, Inc.), 1.0 g of tetraethyl orthosilicate and 3.0 g of 8-glycidoxyoctyltrimethoxysilane were added, and the mixture was ultrasonically dispersed for three hours. After filtering the obtained dispersion, dispersion in ion exchanged water and centrifugation were repeated three times to obtain magnetically responsive beads EP having an epoxy group on the surface. After ultrasonically dispersing the magnetically responsive beads EP in PBS to 3.0 wt%, 0.5 mL was dispensed in a test tube. After trapping with a magnet and removing the supernatant, 1 mL of MU-3 monoclonal antibody (1.07 mg/mL, 150 mM phosphate buffer solution, pH7.8) was added to the magnetic beads and reacted at 25°C for 72 hours under stirring. After washing the magnetic beads, 2 mL of 1% BSA-containing phosphate buffer solution was added, and blocking was conducted at room temperature for one day and one night under stirring. A MU-3 monoclonal antibody-immobilized magnetic bead solution was thus prepared (sometimes abbreviated to "MU-3 antibody beads" below.). The solution was diluted with a bead diluent to a magnetic bead amount of 1 mg/mL before use. The composition of the bead diluent is shown below.

Composition of bead diluent: 0.025 mol/L tris buffer solution (pH7.8), 0.01 mol/L NaCl, 0.025% Tween20, 0.09% NaN₃, 0.5 mMEDTA-2Na, 1% saccharose, bovine serum albumin 0.5%

### <Preparation of Ru-Labeled Anti-Prothrombin F1 Region Monoclonal Antibody Solution>

To 1 mL of an anti-prothrombin F1 region monoclonal antibody (prepared at 1 mg/mL), 68 µL (10 mg/mL) of a Ru complex compound of succinimide group-modified tri-dipyridyl ruthenium was added, and the reaction was advanced under stirring at room temperature for 30 minutes. The reaction was stopped by adding 50 µL of 2 mol/L glycine, and the mixture was further stirred at room temperature for 10 minutes. In the end, the sample was loaded to sephadex G-25 (equilibrated with 10 mmol/L phosphate buffer solution) to collect a Ru-bound protein fraction, and a Ru-labeled anti-prothrombin F1 region monoclonal antibody was thus prepared (sometimes abbreviated to "Ru-labeled anti-Fl antibody" below). The obtained Ru-labeled anti-Fl antibody was diluted with a Ru diluent (2) to a final concentration of 2 µg/mL before use. The composition of the Ru diluent (2) is shown below.

### Composition of Ru diluent (2): 0.05 mol/L HEPES buffer solution (pH7.8), 0.09% NaN₃, 0.2 mg/mL mouse IgG, 0.003 M EDTA-4Na, 0.1% Tween20

### <Measurement of PIVKA-II>

The measurement of PIVKA-II was made using an automated ECLIA assay device "Picolumi III". Standard antigen solutions containing the standard PIVKA-II antigen at 0 mAU/mL, 10 mAU/mL, 100 mAU/mL and 1000 mAU/mL were prepared. Reaction tubes in the number of measurements were prepared, and 100 µL of the reaction solution was dispensed to all the reaction tubes. Each of the standard antigen solutions in a volume of 20 µL was added to two reaction tubes. As the first reaction, 25 µL of the MU-3 monoclonal antibody-immobilized magnetic bead solution was added, and the reaction was advanced at a temperature of 30±2°C for five minutes. The reaction solutions were stirred for several seconds at certain intervals during the reaction. After collecting the beads on the reaction tube wall by bringing a magnet close to a reaction tube, the liquid in the reaction tube was removed by suction. As a washing step, 350 µL of Picolumi BF washing solution (manufactured by Sekisui Medical Co., Ltd.) was added to each reaction tube and stirred, and after collecting the beads on the reaction tube wall by bringing a magnet close to the reaction tube, the liquid in the reaction tube was removed by suction. The washing step was repeated again. Next, as the second step, 100 µL of the Ru-labeled anti-Fl antibody solution was added to the reaction tubes, and the reaction was advanced at a temperature of 30±2°C for three minutes. The reaction solutions were stirred for several seconds at certain intervals during the reaction. As the washing step, 350 µL of BF washing solution was added to each reaction tube and stirred, and after collecting the beads on the reaction tube wall by bringing a magnet close to the reaction tube, the liquid in the reaction tube was removed by suction. The washing step was repeated again. In the end, 300 µL of a luminous electrolyte (manufactured by Sekisui Medical Co., Ltd.) was added to the reaction tubes, and the beads were led to flow cell electrodes, thereby measuring the luminous intensities.

### (Results and Discussion)

The results of measurement of PIVKA-II using antibody 202 alone, antibody 203 alone, antibody 205 alone, antibody 206 alone, a mixture of antibody 202 and antibody 203, a mixture of antibody 202 and antibody 205, a mixture of antibody 202 and antibody 206, a mixture of antibody 203 and antibody 205, a mixture of antibody 203 and antibody 206 and a mixture of antibody 205 and antibody 206 as the Ru-labeled anti-Fl antibodies are shown in Table 2 and Fig. 1.

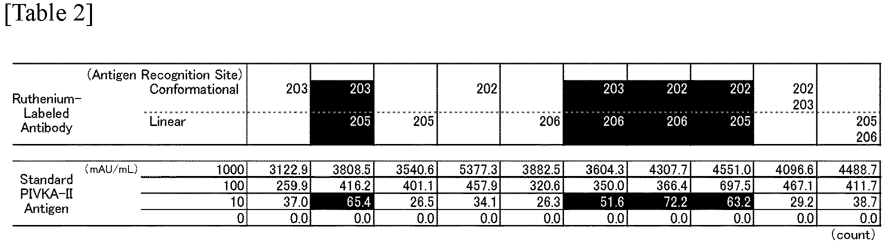

As it is obvious from Table 2 and Fig. 1, when an antibody recognizing a linear epitope (antibody 202 and antibody 203) and an antibody recognizing a conformational epitope (antibody 205 and antibody 206) were mixed and used (indicated with white letters on a black background), significant increases in the measurement value (count number) at 10 mAU/mL were observed.

Although the inventors do not intend to be bound by any particular theory, it is presumed that a part of the F1 region of PIVKA-II (having the same primary sequence as that of the F1 region of prothrombin) is denatured and has lost the native conformation in a reaction solution. It is believed that, when an antibody recognizing a linear epitope and an antibody recognizing a conformational epitope are combined, PIVKA-II contained in the reaction solution may be captured thoroughly.

### INDUSTRIAL APPLICABILITY

The measurement method and the measurement reagent for an analyte using immunoreaction according to the invention can be used for a diagnosis or support of a diagnosis of hepatoma or the like. Moreover, according to the invention, the sensitivity of an existing measurement method and an existing measurement reagent for an analyte using immunoreaction can be improved.

## Claims

1. A sandwich immunoassay method for measuring an analyte using a first antibody(s) and a second antibody(s), wherein one or both of the first antibody(s) and the second antibody(s) are a mixture of a monoclonal antibody recognizing a linear epitope and a monoclonal antibody recognizing a conformational epitope.

2. A sandwich immunoassay method for measuring an analyte in a sample including the following steps:
a step of providing a sample;
a step of bringing a first antibody(s) into contact with the sample and thereby providing a first reactant;
a step of optionally recovering a first antibody-analyte complex contained in the first reactant;
a step of bringing a second antibody(s) into contact with the first reactant or the first antibody-analyte complex and thereby providing a second reactant;
a step of recovering a first antibody-analyte-second antibody complex contained in the second reactant; and
a step of measuring the analyte in the sample by measuring the signal of a label of the recovered first antibody-analyte-second antibody complex;
wherein the first antibody(s) and the second antibody(s) are antibodies for different epitopes of the analyte, and
one or both of the first antibody(s) and the second antibody(s) are a mixture of a monoclonal antibody recognizing a linear epitope and a monoclonal antibody recognizing a conformational epitope.

3. The method according to claim 1 or 2, wherein the first antibody(s) is an immobilized antibody.

4. The method according to any of claims 1 to 3, wherein the second antibody(s) is a labeled antibody.

5. The method according to any of claims 1 to 4, wherein the analyte is PIVKA-II.

6. The method according to any of claims 1 to 5, wherein the monoclonal antibody recognizing a linear epitope is an antibody which recognizes a peptide having the sequence ((E/y)AF(E/y)AL(E/y)SSTATDVFWAKY) of the 26th to 44th amino acids of PIVKA-II or a peptide having the sequence ((E/y)AL(E/y)SSTATDVFWAKY) of the 29th to 44th amino acids of PIVKA-II.

7. The method according to any of claims 1 to 6, wherein the monoclonal antibody recognizing a conformational epitope is an antibody which recognizes the conformation of the amino acid sequence (SSTATDVFWAKYT) of the 33rd to 45th residues, the amino acid sequence (TATDVFWAKYT) of the 35th to 45th residues or the amino acid sequence (TATDVFWA) of the 35th to 42nd residues of PIVKA-II.

8. A sandwich immunoassay reagent for measuring an analyte comprising a first antibody(s) and a second antibody(s), wherein one or both of the first antibody(s) and the second antibody(s) are a mixture of a monoclonal antibody recognizing a linear epitope and a monoclonal antibody recognizing a conformational epitope.


SEQUENCE LISTING

<110>  SEKISUI MEDICAL CO. LTD.

<120>  Immunological analyte measurement method and reagent

<130>  19P01400

<160>  8

<170>  PatentIn version 3.5

<210>  1
<211>  19
<212>  PRT
<213>  Homo sapiens

<400>  1

Glu Ala Leu Glu Ser Ser Thr Ala Thr Asp Val Phe Trp Ala Lys Tyr
1               5                   10                  15

Thr Ala Cys

<210>  2
<211>  19
<212>  PRT
<213>  Artificial Sequence

<220>
<223>  Synthetic peptide

<220>
<221>  MISC_FEATURE
<222>  (1)..(1)
<223>  biotinylated

<400>  2

Glu Ala Phe Glu Ala Leu Glu Ser Ser Thr Ala Thr Asp Val Phe Trp
1               5                   10                  15

Ala Lys Tyr

<210>  3
<211>  19
<212>  PRT
<213>  Artificial Sequence

<220>
<223>  Synthetic peptide

<220>
<221>  MISC_FEATURE
<222>  (1)..(1)

<223>  biotinylated

<220>
<221>  MISC_FEATURE
<222>  (7)..(7)
<223>  Gla residue

<400>  3

Glu Ala Phe Glu Ala Leu Glu Ser Ser Thr Ala Thr Asp Val Phe Trp
1               5                   10                  15


Ala Lys Tyr




<210>  4
<211>  19
<212>  PRT
<213>  Artificial Sequence

<220>
<223>  Synthetic peptide


<220>
<221>  MISC_FEATURE
<222>  (1)..(1)
<223>  biotinylated

<220>
<221>  MISC_FEATURE
<222>  (4)..(4)
<223>  Gla residue

<400>  4

Glu Ala Phe Glu Ala Leu Glu Ser Ser Thr Ala Thr Asp Val Phe Trp
1               5                   10                  15


Ala Lys Tyr




<210>  5
<211>  19
<212>  PRT
<213>  Artificial Sequence

<220>
<223>  Synthetic peptide


<220>
<221>  MISC_FEATURE
<222>  (1)..(1)
<223>  biotinylated

<220>
<221>  MISC_FEATURE
<222>  (4)..(4)

<223>  Gla residue

<220>
<221>  MISC_FEATURE
<222>  (7)..(7)
<223>  Gla residue

<400>  5

Glu Ala Phe Glu Ala Leu Glu Ser Ser Thr Ala Thr Asp Val Phe Trp
1               5                   10                  15

Ala Lys Tyr

<210>  6
<211>  19
<212>  PRT
<213>  Artificial Sequence

<220>
<223>  Synthetic peptide

<220>
<221>  MISC_FEATURE
<222>  (1)..(1)
<223>  biotinylated

<400>  6

Glu Ala Leu Glu Ser Ser Thr Ala Thr Asp Val Phe Trp Ala Lys Tyr
1               5                   10                  15

Thr Ala Cys

<210>  7
<211>  10
<212>  PRT
<213>  Artificial Sequence

<220>
<223>  Synthetic peptide

<220>
<221>  MISC_FEATURE
<222>  (1)..(1)
<223>  biotinylated

<400>  7

Thr Ala Thr Asp Val Phe Trp Ala Lys Tyr
1               5                   10

<210>  8
<211>  13

<212>  PRT
<213>  Artificial Sequence

<220>
<223>  Synthetic peptide

<220>
<221>  MISC_FEATURE
<222>  (1)..(1)
<223>  biotinylated

<400>  8

Thr Ala Thr Asp Val Phe Trp Ala Lys Tyr Thr Ala Cys
1               5                   10